(19) Europäisches Patentamt
European Patent Office
Office européen des brevets

(11) Veröffentlichungsnummer: **0 095 637**
**A2**

(12) # EUROPÄISCHE PATENTANMELDUNG

(21) Anmeldenummer: **83104788.1**

(22) Anmeldetag: **16.05.83**

(51) Int. Cl.³: **C 07 D 239/30**, C 07 D 241/44

(30) Priorität: **28.05.82 DE 3220105**

(43) Veröffentlichungstag der Anmeldung: **07.12.83**
**Patentblatt 83/49**

(84) Benannte Vertragsstaaten: **CH DE FR GB LI**

(71) Anmelder: **BAYER AG, Zentralbereich Patente, Marken und Lizenzen, D-5090 Leverkusen 1, Bayerwerk (DE)**

(72) Erfinder: **Werner, Friedrich, Dr. Bayer do Brasil S.A., Estrada Boa Esperanza, s/n, 26150 Belford Roxo (BR)**
Erfinder: **Mannes, Karl, Dr., Kurt-Schumacher-Ring 119, D-5090 Leverkusen 1 (DE)**

(54) **Verfahren zur Herstellung von in alpha-Stellung chlorierten Stickstoff-Heterocyclen.**

(57) Es wird ein neues Verfahren zur Herstellung von in α-Stellung chlorierten Stickstoff-Heterocyclen bereitgestellt.

Die Umsetzung erfolgt durch Chlorierung der entsprechenden Hydroxyverbindungen mit Phosgen in Gegenwart einer organischen Phosphorverbindung.

EP 0 095 637 A2

BAYER AKTIENGESELLSCHAFT 5090 Leverkusen, Bayerwerk

Zentralbereich
Patente, Marken und Lizenzen Er/ABC

Verfahren zur Herstellung von in α-Stellung chlorierten Stickstoff-Heterocyclen

Die vorliegende Erfindung betrifft ein neues Verfahren zur Herstellung von Stickstoff-Heterocyclen, die in α-Stellung chloriert sind, durch Umsetzung der entsprechenden Hydroxyverbindungen mit Phosgen in Gegenwart einer organischen Phosphorverbindung.

Gemäß DE-AS 11 64 418 wird diese Herstellung von in α-Stellung chlorierten N-Heterocyclen durch Phosgenierung in Gegenwart substituierter Carbonsäureamide vorgenommen.

Gemäß US-PS 3 282 941 kann die Herstellung von Dichlorchinoxalincarbonsäurechloriden durch Phosgenierung von Dihydroxychinoxalincarbonsäure in Gegenwart von substituierten Carbonsäureamiden erfolgen. Als günstigste Katalysatoren dieses Typs haben sich die niederen Alkylformamide erwiesen. Besonders bevorzugt ist die Verwendung von Dimethylformamid. Nun ist aber bekannt geworden, daß aus niederen Formamiden, beispielsweise aus Dimethylformamid, unter dem Einfluß von chlorierenden Agentien, Carbamoylchloride entstehen (Isw. Akad. Nauk USSR, Chem. Ser. 1971 (3) 513 - 519, zitiert in C.A. 75, 48 340 m), die sich im Tierversuch als krebserregend erwiesen haben (J. Nat. Cancer Int. 48 (5), 1539 - 1541 (1972)). Deshalb erfordert die Verwendung dieser Katalysatoren einen deutlich erhöhten sicherheitstechnischen Aufwand.

Es wurde nun gefunden, daß die Herstellung von in α-Stellung chlorierten Stickstoffheterocyclen durch Umsetzung der entsprechenden α-Hydroxylverbindungen mit Phosgen dann sehr günstig gelingt, wenn man die Umsetzung in Gegenwart einer organischen Phosphorverbindung durchführt.

Aus der Reihe der für die erfindungsgemäße Umsetzung geeigneten organischen Phosphorverbindungen seien folgende Verbindungsklassen beispielhaft genannt:

Alkyl- und Arylphosphine, Alkyl- und Arylphosphinoxyde, Alkyl- bzw. Arylphosphite und die entsprechenden Phosphate.

Bevorzugte Katalysatoren sind Arylphosphine bzw. Arylphosphinoxyde. Als Einsatzmenge sind 0,1 - 50 Molprozent organischer Phosphorverbindung pro Mol zu chlorierender Ausgangsverbindung (Hydroxyverbindung) geeignet, bevorzugt ist die Verwendung von 0,5 - 4 Molprozent.

Die Reaktion wird vorzugsweise in einem inerten organischen Lösungsmittel durchgeführt. Hierzu eignen sich z.B. Xylol, Chlorbenzol, Dichlorbenzol, Trichlorbenzol, Isododecan und Tetrachlorethan. Es ist aber auch möglich, bei flüssigen Ausgangsprodukten in Substanz zu arbeiten bzw. bei flüssigen Endprodukten diese als Lösungsmittel zu verwenden.

Das erfindungsgemäße Verfahren wird im allgemeinen bei Temperaturen von 120-250°C durchgeführt. Bevorzugt sind Temperaturen von 130-220°C und insbesondere bevorzugt 140-200°C.

Das Verfahren wird im allgemeinen bei Normaldruck bzw. leicht erhöhtem Druck durchgeführt. Es ist aber ebenso möglich, bei vermindertem bzw. stark erhöhtem Druck zu arbeiten.

Im allgemeinen wird die Reaktion folgendermaßen durchgeführt:

Die zu chlorierende Hydroxy-Verbindung wird mit dem Katalysator in das Lösungsmittel eingebracht und die Mischung bzw. Suspension auf Reaktionstemperatur geheizt. Dann wird Phosgen in die Mischung eingeleitet bis der Umsatz vollständig ist. Hierzu sind üblicherweise 1-3 Mol Phosgen pro auszutauschender OH-Gruppe erforderlich. Zur Aufarbeitung wird das Produkt entweder durch Kristallisation direkt isoliert oder man destilliert das Lösungsmittel vollständig ab und reinigt dann gegebenenfalls die chlorierte Stickstoffheterocyclische Verbindung durch Destillation, Sublimation oder Kristallisation. Für die erfindungsgemäße Umsetzung sind als zu chlorierende hydroxygruppenhaltige Stickstoffheterocyclen besonders geeignet: 4-Methyl-5-chloruracil; 4-Methyluracil; 2,3-Dihydroxy-chinoxalin-6-carbonsäure; 2,3-Dihydroxy-chinoxalin; 2,3-Dihydroxy-6-methyl-chinoxalin.

Es muß als außergewöhnlich überraschend angesehen werden, daß mit den genannten Katalysatoren Reaktionen auch oberhalb von 150°C mit guten Ausbeuten durchführbar sind. Dies war nach dem Stand der Technik (US-Patent 3 282 941 S. 2 Zeile 51 folgende ) nicht zu erwarten.

Die erfindungsgemäß hergestellten Verbindungen sind für verschiedene Verwendungszwecke geeignet. Sie können z.B. mit entsprechenden chromophoren Systeme in an sich bekannter Weise zu Reaktivfarbstoffen umgesetzt werden:

z.B.

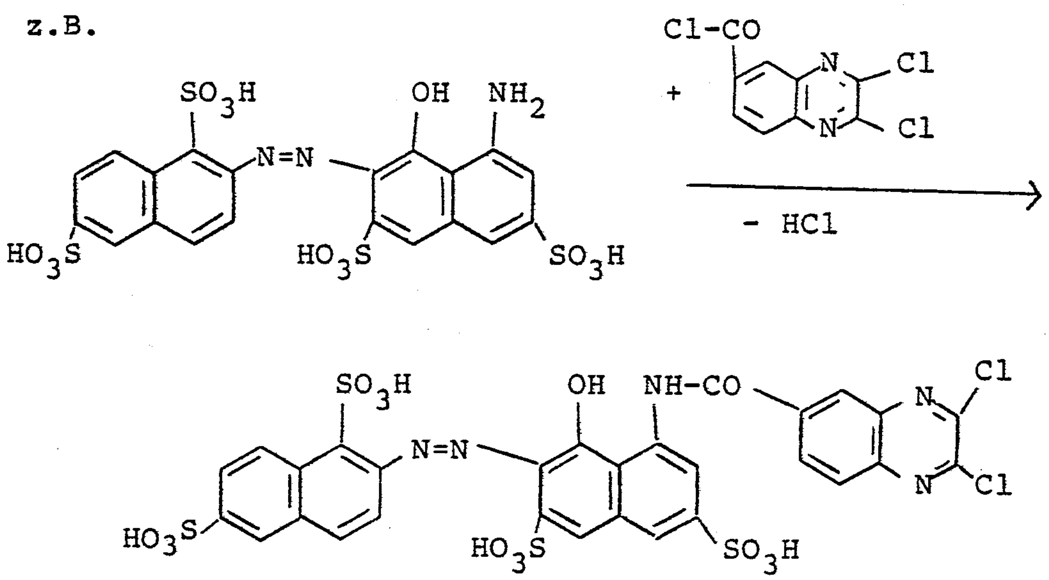

Beispiel 1

523,5 g 4-Methyl-5-chloruracil 92 %ig = 481,6 g 100 %ig werden in 800 ml o-Dichlorbenzol suspendiert. Zur Lösung gibt man 15 g Triphenylphosphin und heizt auf 170°C. Unter Rühren werden 1275 g Phosgen in 40 Stunden mit 42,5 g/h gegen 300 mm Wassersäule eingeleitet. Dabei wird die Suspension zur dunklen Lösung. Anschließend wird entgast und o-Dichlorbenzol im steigenden Vakuum abdestilliert bis die Sumpftemperatur bei 20 mbar 180°C erreicht. Man gewinnt 1617 g Destillat mit einem Gehalt von 33,0 % = 533,6 g 6-Methyl-2,4,5-trichlorpyrimidin, entsprechend 90,0 % d.Th. (aus Gaschromatographie). Durch eine Kolonnendestillation kann 6-Methyl-2,4,5-trichlorpyrimidin vom o-Dichlorbenzol abgetrennt und rein isoliert werden.

Beispiel 2

In eine Suspension von 128,7 g 4-Methyluracil in 400 ml o-Dichlorbenzol werden 5 g Triphenylhosphin gegeben. Es wird auf 170°C geheizt und Phosgen mit 35 g/h gegen 300 mm Wassersäule eingeleitet. Nach 10 Stunden wird die Phosgenierung beendet. Es wird entgast und dann o-Dichlorbenzol und 6-Methyl-2,4,5-dichlorpyrimidin im steigenden Vakuum abdestilliert bis die Sumpftempratur bei 20 mbar 180°C erreicht. Man erhält 661,1 g Produkt, die zu 79,0 % aus O-Dichlorbenzol und 21,0 % = 138,8 g 6-Methyl-2,4,5-dichlorpyrimidin, entsprechend 85,2 % d.Th., bestehen.

0095637

Beispiel 3

460 g 2,3-Dihydroxychinoxalin-6-carbonsäure 92,3 %ig = 424,6 g 100 % (Produkt enthält ca. 7 % Wasser) werden in 800 ml o-Dichlorbenzol suspendiert und mit 10,6 g Triphenylphosphin versetzt und unter Rühren auf 130°C geheizt. Dann wird Phosgen mit 90 g/h eingeleitet. Nach 5 Stunden wird die Temperatur auf 150°C gesteigert und weitere 11 Stunden Phosgen eingeleitet. Man phosgeniert weitere 2 Stunden mit je 15 g Phosgen, wonach die Phosgenaufnahme beendet ist. Es wird 1 Stunde gerührt und 1 h restliches Phosgen mit Stickstoff ausgeblasen. Man kühlt auf 100°C und destilliert im steigenden Wasserstrahlvakuum o-Dichlorbenzol ab bis die Sumpftemperatur bei 20 mbar 140°C erreicht. Man gewinnt 785 ml o-Dichlorbenzol und 565 g 2,3-Dichlorchinoxalin-6-carbonsäurechlorid roh 85,6 %ig (HFC-Analytik) = 89,8 % d.Th.

Beispiel 4

Die Einsätze und die Ausführungsweise sind analog Beispiel 3. Als Kaltalysator werden 11,20 g Triphenylphosphinoxyd eingesetzt. Die Ausbeute beträgt 560 g 2,3-Dichlorchinoxalin-6-carbonsäurechlorid roh 86,3 %ig = 89,7 % d.Th.

Beispiel 5

88 g 2,3-Dihydroxy-6-methylchinoxalin (0,5 Mol) werden in 250 ml o-Dichlorbenzol suspendiert. Man gibt 2,6 g Triphenylphosphin zum Ansatz und heizt auf 170°C. Bei dieser Temperatur beginnt man mit dem Einleiten von ca. 20 g Phosgen pro Stunde. Nach Einleiten von

0095637

insgesamt 150 g Phosgen ist eine klare Lösung entstanden.

Das Reaktionsgemisch wird eine Stunde bei 170°C nachgerührt, entgast und anschließend im Vakuum bis zum Erreichen einer Sumpftemperatur von 150°C bei 20 mbar eingedampft. Dabei wird das o-Dichlorbenzol fast quantitativ zurückgewonnen. Als Rückstand erhält man 102 g Rohprodukt mit einem Gehalt von 90,1 %, entsprechend 91,9 g 2.3-Dichlor-6-methylchinoxalin. Das sind 86,3 % der Therorie.

Das Rohprodukt läßt sich durch Sublimation leicht weiter reinigen.

Beispiel 6

81 g 2.3-Dihydroxychinoxalin werden in Gegenwart von 2,6 g Triphenylphosphin in o-Dichlorbenzol analog der Verfahrensweise des Beispiels 5 phosgeniert.

Man erhält 113 g rohes 2.3-Dichlorchinoxalin mit einem Gehalt von 83,3 %, das sich ebenfalls durch Sublimation leicht reinigen läßt.

Patentansprüche

1. Verfahren zur Herstellung von Stickstoff-Heterocyclen, die in $\alpha$-Stellung chloriert sind, durch Umsetzung der entsprechenden Hydroxyverbindung mit Phosgen, dadurch gekennzeichnet, daß man die Umsetzung in Gegenwart einer organischen Phosphorverbindung durchführt.

2. Verfahren nach Anspruch 1, dadurch gekennzeichnet, daß man die Umsetzung in einem organischen Lösungsmittel durchführt.

3. Verfahren nach Anspruch 1 bis 2, dadurch gekennzeichnet, daß man die Umsetzung bei Temperaturen von 120 bis 250°C durchführt.

4. Verfahren nach Anspruch 1 bis 3, dadurch gekennzeichnet, daß man als organische Phosphorverbindung Triphenylphosphin und/oder Triphenylphosphinoxyd einsetzt.

5. Verfahren nach Anspruch 1 bis 4, dadurch gekennzeichnet, daß man pro Mol zu chlorierender Hydroxyverbindung 0,5 bis 4 Molprozent organischer Phosphorverbindung einsetzt.

6. Verfahren nach Ansprüchen 1 bis 5, dadurch gekennzeichnet, daß man pro auszutauschender OH-Gruppe der Hydroxyverbindung 1 bis 3 Mol Phosgen einsetzt.

7. Verfahren nach Anspruch 1 bis 6, dadurch gekennzeichnet, daß man als zu chlorierenden Hydroxyverbindung eine Verbindung der Reihe 4-Methyl-5-chloruracil; 4-Methyluracil; 2,3-Dihydroxy-chinoxalin-6-carbonsäure; 2,3-Dihydroxy-6-methylchinoxalin; 2,3-Dihydroxychinoxalin einsetzt.